Europäisches Patentamt

European Patent Office

Office européen des brevets

(11) Publication number: **0 318 246**
**A2**

## EUROPEAN PATENT APPLICATION

(21) Application number: 88311037.1

(22) Date of filing: 22.11.88

(51) Int. Cl.⁴: **G21F 9/00**

(30) Priority: 23.11.87 NO 874874

(43) Date of publication of application:
**31.05.89 Bulletin 89/22**

(84) Designated Contracting States:
**AT BE CH DE ES FR GB GR IT LI LU NL SE**

(71) Applicant: **MATFORSK NORSK INSTITUTT FOR NAERINGSMIDDELFORSKNING**
**Osloveien 1 P.O. Box 50**
**N-1432 As(NO)**

(72) Inventor: **Aukrust, Lars**
**Naeringsmiddelforskning**
**Osloveien 1**
**N-1430 As(NO)**

(74) Representative: **Goldin, Douglas Michael et al**
**J.A. KEMP & CO. 14, South Square Gray's Inn**
**London WC1R 5EU(GB)**

(54) Process and means to avoid absorption of harmful compounds.

(57) Device to prevent assimilation of harmful/unwanted material in the digestive tract, said device comprising disperse particles, optionally monodisperse spheres, which are not assimilated or penetrate the walls in the digestive tract, and to which particles there is attatched or bound one or more binding/immobilizing materials for the harmful/unwanted matter. Said particles may be taken in together with food containing the harmful or unwanted matter, for instance radionuclides or heavy metals.

EP 0 318 246 A2

The present invention concerns a method for selective prevention of assimilation of harmful compounds in the organism by digestion of food products containing such compounds, and a device for performing such a method.

The principle behind the present invention lies in using on inert particles bound/immobilized compounds which in their turn specificly can bind/immobilize such harmful/improper compounds in the digestive tract. As an example such harmful compounds may comprise radionuclids, heavy metals, carsinogens, toxins or other ahrmful or irritating compounds which may be absorbed by the digestive system. Such material may exsist in any form of foodstuff or nutritious material either in solid or liquid form.

There is previously known that indigestable capsules liberating medical compounds or corresponding capsules containing binders for harmful matter may be given to ruminants for permanent or intermittant residence in the digestive system. Such capsules have, however, the disadvantage that they have only stationary effect in a small part of the digestive system.

There is also previously known that specific compounds in finely divided form in the digestive tract are able to bind harmful compounds which thereby is prevented from being absorbed and which instead are removed via feces. The advantage with these compounds is that they have a relatively large actove surface and that they are distributed in the entire digestive tract. The disadvantages are, however, diverse, namely that they have

(1) an undefined particle size, which may have the consequence that the smallest particles are absorbed by the intestinal cells,

(2) an unspecific binding capacity (f.ex. active charcoal),

(3) are not always completely insoluble, and

(4) may in themselves be toxic.

The present invention is employing the discovery the spheical particles with a certain size distribution (f.ex. so-called "Dyno$^R$-spheres", which may be monodisperse) are distributed evenly throughout the digestive tract without being absorbed by the intestinal cells. It has also been shown that the material comprising such particles is not toxic, and that it is not broken down in the digestive system of humans.

By in a firm, irreversible manner (f.ex. covalently) coupling such spheres to a material which in turn selectively can bind/immobilize the compounds in re which is to be removed, food or foodstuffs containing such harmful matter may be taken in together with the material removing such compounds according to the invention, without the organism assimilating the harmful compounds, since such harmful matter is immobilized/bound at any point throughout the digestive tract and is expelled together with feces.

Examples of binding material are ligand groups common in metal complexes, different kinds of ion exchange compounds such as chelates (EDTA, DTPA, iminodiacetate etc.) and compounds organized in a space lattice (Prussian Blue, zeolite etc.), or selective binders for organic compounds (antibodies, enzymes etc.).

According to the present invention the selectively binding material may optionally be present on the surface of the carrier spheres or also inside these, provided they are permeable for the compound which is to be removed. Of course there may also be present different binders on the carrier spheres, or the disperse material may consist of a mixture of spheres with differently binding material attatched.

The present invention may for example be used to prevent animals from absorbing radioactive cesium from foodstuffs both for animals and humans, to prevent absorption of heavy metals in farm animals, or for treatment of acute poisoning where poison from mushrooms, bacteria or other sources are involved.

Below, there is, as an examole, presented a test for a material according to the invention using Prussian Blue immobilized on inert plastic spheres to prevent assimilation of radio cesium in rats fed with a diet containing radioactively polluted meat from reindeer.

Meat from reindeer with a too high content of cesium-134 + cesium-137 (34 970 Bq/kg) was homogenized, fries without additives i a teflon frying pan, homogenized and mixed in the weight ratio 4 : 1 with pulverized rat feed (R3, Ewos AB, Sweden). Female Wistar rats, 150-160g from Moellegaard Hansens Breeding Laboratories, Copenhagen, Denmark, were used in metabolic cages. Inert polymer spheres with immobilized Prussian Blue was purchased from prof. Giese, Bishofholer Damm 15, D-3000 Hannover, Germany. A fraction of the spheres, 0,3 - 0,5 mm diameter, was separated and used in the tests. The activity of radioactive cesium (cesium-134 + cesium-137) was measured in a gamma scintillation detector from Nuclear Enterprises Ltd., Edinburgh, Scotland.

Measurements of the radioactivity from the whole body was performed by placing the detector precisely over the back of rats being encaged in a plastic tube with an inner diameter of 55mm.

Polymer spheres with Prussian Blue were given via gavage using destilled water of a total volume of 1,5 - 2 ml.

Test 1.

Rats were fed individually in metabolic cages with the radiocesium-containing diet two nights in a row. In the evening and in the morning six rats received 500 mg Prussian Blue spheres, whereas six rats received 1,5-2 ml detilled water. All animals were given water ad libitum.

Test 2.

Rats were fed individually with the radiocesium-containing diet in two 35-minutes periods per 24 hours: One in the morning and one in the evening. The test continued for a total of five successive such periods (meals). Three of the animals received approx. 10 min. after each meal 300 mg of Prussian Blue speres according to the invention. Three control animals did not receive any spheres, but were otherwise treated similarly. The light periods per 24 hours were turned, so that it was bright in the night-time in the test room. The rats were accustomed to this rythm in the light/dark periods during two days immediately prior to the commencement of the test. They were given water ad libitum. At the end of each dark period each rat received 5 g of standard rat feed in the form of pellets.

Reults:

Test 1:

The rats ate (mean valus ± standard deviations) $26,2 \pm 8,2$ g and $29,2 \pm 4,0$ g of radioactive feed respectively per animal in the test- and control groups, corresponding to a consumption of radiocesium of (range) 513 - 1434 Bq and 984 - 1376 Bq per animal respectively. The whole body radiocesium activity in percent of the total consumed activity day 1 - 3 are presented in table 1 (corrected for background activity day 1).

Table 1

| (Mean ± standard deviation, n = 6.) | | | | |
|---|---|---|---|---|
| | Day 2 | Day 3 | Day 4 | Day 5 |
| Control animals | 36,6±9,4 | 78,9±3,5 | 70,3±5,8 | 66,2±5,5 |
| Test animals | 41,3±7,3 | 78,0±4,0 | 53,1±4,6 | 49,7±6,5 |
| (% of controls) | (113) | (99) | (76) | (75) |

From table 1 it is evident that there is no difference in whole body activity between the test and control animals during the days (1-3) they were given radioactive feed. The test animals have, however, a reduced activity during the successive days compared to the control animals.

Test 2.

The rats ate radioactivity corresponding to a total of (range) 689 - 1281 Bq and 646 - 1086 Bq per animal for the test and control animals respectively during the five meals. The whole body activity of radiocesium corrected for background day 1 is shown in table 2 in percent of total consumed activity day 1-3.

Table 2

| (Mean ± standard deviation, n = 3.) | | | | |
|---|---|---|---|---|
| | Day 2 | Day 3 | Day 4 | Day 5 |
| Control animals | 55,4±6,2 | 64,6±4,7 | 65,1±3,2 | 63,1±7,7 |
| Test animals | 35,8±15,8 | 36,3±6,8 | 21,5±5,8 | 15,2±3,2 |
| (% of controls) | (65) | (56) | (33) | (24) |

From table 2 it is evident that there continuously is less whole body activity in the test animals also during the days they are given radioactive feed. Day 5 the test animals have left only 15% of the activity they were given through the feed, which is one quarter of the remaining activity in the control animals.

The tests prove that Prussian Blue immobilized on inert polymer spheres have a great ability to reduce the absorption of radiocesium in rats when the administration is done directly in connection with a meal containing radioactive reindeer meat. The reduction was 76% compared to a control group. When the radioactive feed was given during full nights and the Prussian Blue spheres were given before and after this period, the reduction in assimilation became significantly less, only 25%.

The above given tests are given as example only, and should not be construed as limiting to the scope of the invention.


**Claims**

1. Device for preventing assimilation of harmful substances from the digestive tract, **characterized in** that it comprises disperse particles onto which there is attatched/bound one or more binding/immobilizing materials specific for the harmful substances, which particles are of such a character that they do not penetrate the walls in the digestive tract or are destroyed/decomposed by the chemical and physical conditions in the digestive tract.

2. Device according to claim 1, **characterized in** that the disperse particles are monodisperse spheres.

3. Device according to claim 1 or 2, **characterized in** that more than one selectively binding material is bound to the particles/spheres.

4. Device according to claims 1 - 3, **characterized in** that a mixture of particles/spheres is attatched to different types of binding materials.

5. Process for preventing assimilation of harmful/unwanted material from foodstuff in the digestive tract, **characterized in** that a material according to claims 1 - 4 is taken in together or in direct connection with a meal containing the harmful/unwanted material.

6. Use of device according to claims 1 - 4 to prevent assimilation of radionuclides in the digestive tract.

7. Use of device according to claims 1 - 4 to prevent assimilation of heavy metals in the digestive tract.